# EUROPEAN PATENT SPECIFICATION

(11) **EP 3 254 632 B1**
(45) Date of publication and mention of the grant of the patent: **12.12.2018**
(21) Application number: 17181918.8
(22) Date of filing: 27.03.2009
(51) Int. Cl.: A61B 17/34, A61M 16/16, B05B 17/06, A61M 15/00

(54) **HUMIDIFICATION IN BREATHING CIRCUITS**
BEFEUCHTUNG IN ATEMKREISLÄUFEN
HUMIDIFICATION DE CIRCUITS RESPIRATOIRES

(30) Priority: 28.03.2008 IE 20080243; 26.09.2008 IE 20080778
(43) Date of publication of application: 13.12.2017
(62) Divisional of application: 09725321.5
(73) Proprietor: Stamford Devices Limited, Dangan, Galway (IE)
(72) Inventor: CLANCY, Dermot Joseph, County Leitrim (IE); DUFFY, Conor, County Galway (IE); GIBBONS, Keith, Galway (IE); POWER, John, County Galway (IE); SMITH, Niall, Scotland FR10 1RR (GB)
(74) Representative: O'Brien, John Augustine

(56) References cited:
- WO-A-2004/074791
- US-A- 5 918 593
- US-A- 5 938 117
- US-A- 6 014 970
- US-A1- 2003 196 660
- US-A1- 2006 151 624

## Description

### Introduction

This invention relates to humidification in breathing circuits for intensive care management of mechanically ventilated patients.

During a normal 24 hour period 250-350 ml of water is lost from the respiratory tract.

During normal breathing the upper respiratory tract humidifies and filters the inspired air. This task occurs primarily in the nasopharynx where air is exposed to a large area of highly vascular, moist mucus membrane. On exhalation some of the moisture taken to humidify the air during inspiration is recovered but the balance, the 250-350ml, is replaced from systemic reserves over time. However, following intubation of patients on mechanical ventilation these normal upper airway moisture exchanging structures are bypassed and the burden of moistening the gases is passed to the lower respiratory tract, which is not suited to this task

A ventilator is used to mechanically move breathable air into and out of patients lungs. For patients who have a long term dependence on a ventilator an endotracheal tube (ET) is passed directly into the patient's trachea in order to ensure that air is able to reach the lungs. The ET is connected by tubing to a Y shaped junction, known as a wye which is connected to the ventilator. One limb of the wye is active during an inspiration phase during which air is delivered into the lungs and the other limb is active during an exhalation phase during which exhaled air is expelled from the lungs.

Heat and moisture exchange (HME) devices are often placed in breathing circuits. They extract heat and moisture from humidified gas exhaled by a patient during the exhalation phase and use the extracted heat and moisture to humidify the dry inspiration gas from the ventilator.

In passive HME devices exhaled heat and humidity is absorbed and transferred to inhaled gas. Such passive heat and moisture exchange systems generally at best only recover 70% of exhaled humidity, creating a humidity deficit, which can create thick obstructive secretions and inflammatory airway reactions in patients with chronic airways disease. Consequently, passive HME is largely confined to use with patients with relatively healthy lungs.

It is also known to augment passive HME devices with the addition of heat and water to boost inhaled absolute humidity. In one known system there is direct application of water to the HME element, and heat is applied to the HME element. In another known system water is heated and vapour passes through a Goretex membrane between the HME device and the patient airway.

In a third known system an active heated humidifier is used in which heat is applied to a body of water, and gas from the ventilator is passed over or through the water as the gas passes along the circuit to the patient, adding water vapour to the gas. Such systems may include heated wires in the inspiratory limb of the ventilator tubing to add heat, or reduce heat loss of gas in transit to the patient.

Some of the problems with known active heated water humidifier systems are that the heated humidifier is bulky, heavy and must be placed close to the ventilator distal to the patient. In addition, there is a likelihood of rain-out down the ventilator circuit tubing which can block the tubing, saturate the exhalation filter or flood the patient. As heated humidified gas passes through the inspiratory limb of the ventilator circuit, it cools and water condenses in the tubing. This condensate can obstruct the airway, interfere with ventilation and/or increase the growth of pathogens. These problems can be mitigated by heating the condensate with heated wires but such heated wire systems add to cost and complexity.

In summary active HME systems add expense with only marginal benefits in humidity. In addition, they add bulk and weight at the airway.

This invention is directed towards providing humidification in breathing circuits which will address at least some of these problems.

US2003/0196660A describes a ventilator circuit including a nebuliser.

### Statements of Invention

The invention is defined in the claims.

In one embodiment the ventilator circuit comprises an endotracheal tube, an inspiration line extending from a ventilator and an exhalation line extending from the ventilator.

The method may comprise the step of controlling the aerosolisation. Accurate control may be achieved via high speed pulsing of the electrical signal to the aerosol generator. Accurate changes to the pulse frequency will produce accurate and repeatable changes in aerosol output.

Aerosolisation may be controlled responsive to the flow of ventilation gas in the inspiration line. Greater volumes of gas require proportionally greater fluid flow rate of aerosolised humidifying agent to achieve 100% saturation at a given temperature. The gas flow is measured (via flow or differential pressure sensor) in order to determine and deliver the required volume of humidifying agent to achieve 100% RH. Rapid and accurate measurement of gas flow enables required responsiveness.

The method may comprise controlling the fluid flow rate of the aerosolised humidifying agent.

In one embodiment the method comprises the step of determining the flow rate of ventilation gas in the inspiration line.

In one case the method comprises the step of determining if the humidifying agent is in contact with an aerosol generator. This may involve determining at least one electrical characteristic of the aerosol generator. In one case at least electrical characteristics of the aerosol generator over a range of vibration frequencies is determined.

The method may comprise s the step of comparing the at least one electrical characteristic against a pre-defined set of data.

In one case a humidity meter is included close to the patient to measure the level of humidification of the gas entering the body. In this case a feedback loop to the controller is possible to control output from the nebulizer so as to ensure sufficient humidity is present in the ventilator gas.

In another embodiment the ventilator circuit comprises an inspiration line and an exhalation line which are connected at a junction, and a patient line extending from the junction for connection to an endotracheal tube, and the method comprises the step of delivering the aerosolised humidifying agent into the patient line between the junction and the endotracheal tube.

In one case the ventilation circuit comprises a heat and moisture exchange unit in the patient line and the method comprises the step of delivering the aerosolised humidifying agent into the patient line between the heat and moisture exchange unit and the endotracheal tube.

The ventilation circuit can comprise a heat and moisture exchange unit in the patient line and the method comprises delivering the aerosolised humidifying agent into the heat and moisture exchange unit (HME).

In one arrangement a nebuliser is used to provide humidity at patient side of HME in order to replace humidity losses to allow patients use the HME for longer periods of time.

Alternatively a nebuliser may be integrated with HME ('HME booster') providing humidity to a ventilated patient for longer periods of time possible with conventional HME.

In one embodiment the inspiratory gas is heated by providing a heating means such as a heated wire with the nebulizer providing the humidity. In this case a separate HME or hot pot would not be required.

In one embodiment the aerosolised humidifying agent is delivered into the heat and moisture exchange unit on the patient side of the heat and moisture exchange unit.

In one case the aerosol generator is mounted to the heat and moisture exchange unit.

In one embodiment the aerosol generator is integral with the heat and moisture exchange unit.

The disclosure also provides an aerosol introducer for introducing aerosolised humidifying agent into a ventilation circuit comprising an endotracheal tube, an inspiration line extending from a ventilator, and an exhalation line extending from the ventilator, the introducer comprising an aerosol generator and control means for controlling the operation of the aerosol generator wherein the aerosol generator comprises a vibratable member having a plurality of apertures extending between a first surface and a second surface thereof.

In one case the ventilator circuit comprises an inspiration line and an exhalation line which are connected at a junction, and a patient line extending from the junction comprising e.g. an endotracheal tube, and the method comprises the step of locating the humidifying agent between the junction and the endotracheal tube, or in the inspiratory line prior to the junction.

In one embodiment the controller is configured to control operation of the aerosol generator responsive to the flow of gas in the inspiration line.

In one case the controller is configured to control the flow rate of the humidifying agent to be aerosolised.

In one embodiment the apparatus comprises a device to determine the fluid flow rate of the gas in the inspiration line. The determining device may comprise a flow rate sensor.

In one embodiment the ventilation circuit comprises a junction for connecting the inspiration line and the exhalation line and a patient line for extending between the junction and the endotracheal tube and wherein the aerosol generator is arranged for delivery of aerosolised humidifying agent into the patient line between the junction and the endotracheal tube.

The ventilation circuit may comprise a heat and moisture exchange unit for location in the patient line.

In one arrangement the aerosol generator is arranged for delivery of aerosolised humidifying agent into the patient line between the heat and moisture exchange unit and the endotracheal tube.

The aerosol generator may be mounted on a connector for connection in the patient line.

In one case the aerosol generator is mounted to the heat and moisture exchange unit.

In one embodiment the aerosol generator is integral with the heat and moisture exchange unit.

In one case the first surface is adapted to receive the humidifying agent to be aerosolised.

The aerosol generator may be configured to generate an aerosol at the second surface.

In one case the vibratable member is dome-shaped in geometry. It may also be flat with appropriate stretching.

The vibratable member may comprise a piezoelectric element.

In one case the apertures in the vibratable member are sized to aerosolise the humidifying agent by ejecting droplets of the water such that the majority of the droplets by mass have a size of less than 5 micrometers.

The apertures in the vibratable member can be sized to aerosolise the humidifying agent by ejecting droplets of the water such that the majority of the droplets by mass have a size of less than 3 micrometers.

In one case the controller is configured to control the pulse rate at a set frequency of vibration of the vibratable member.

In one embodiment the controller is impedance matched to the aerosol generator.

In another embodiment the apparatus comprises means to determine whether the humidifying agent is in contact with the aerosol generator.

The determining means may be configured to determine at least one electrical characteristic of the aerosol generator.

The determining means can be configured to determine at least one electrical characteristic of the aerosol generator over a range of vibration frequencies.

In one case the determining means is configured to compare the at least one electrical characteristic against a pre-defined set of data.

### Brief Description of the Drawings

The invention will be more clearly understood from the following description of some embodiments thereof, given by way of example only, with reference to the accompanying drawings, in which: -
Fig. 1A is a diagram of a delivery system according to the disclosure;
Fig. 1B is a perspective view of an apparatus for humidifying gas in a ventilator circuit according to the present disclosure;
Fig. 2 is a schematic illustration of a part of an apparatus according to the disclosure;
Fig. 3 is a schematic illustration of a part of the apparatus of Fig. 1;
Fig. 4 is an exploded isometric view of an aerosol generator used in the disclosure;
Fig. 5 is a cross-sectional view of the assembled aerosol generator of Fig. 4;
Fig. 6 is a perspective view of a controller housing used in the apparatus of the disclosure;
Figs. 7(a) and 7(b) are graphs of DC voltage versus time and AC voltage versus time respectively to achieve a 100% aerosol output;
Figs. 8(a) and 8(b) are graphs of DC voltage versus time and AC voltage versus time respectively to achieve a 50% aerosol output - Fig 8(a) illustrates the waveform output from a microprocessor to a drive circuit and Fig 8(b) illustrates the waveform output from a drive circuit to a nebuliser;
Figs. 9(a) and 9(b) are graphs of DC voltage versus time and AC voltage versus time respectively to achieve a 25% aerosol output - Fig 9(a) illustrates the waveform output from a microprocessor to a drive circuit and Fig 9(b) illustrates the waveform output from a drive circuit to a nebuliser;
Fig 10 is a graph of AC voltage versus time; and illustrates an output waveform from a drive circuit to a nebuliser;
Fig. 11 is a graph of frequency versus current for another apparatus according to the disclosure;
Fig. 12 is a perspective view of another apparatus of the disclosure;
Fig. 13 is a cross sectional view of an apparatus according to the invention;
Fig. 14 is a perspective view of another apparatus of the disclosure; and
Fig. 15 is a perspective view of another apparatus.

### Detailed Description

The invention provides an apparatus for humidifying gas in a ventilator circuit. In the invention a humidifying agent (sterile water or sterile saline) is aerosolised and then delivered to a ventilator circuit coupled to the respiratory system of a patient.

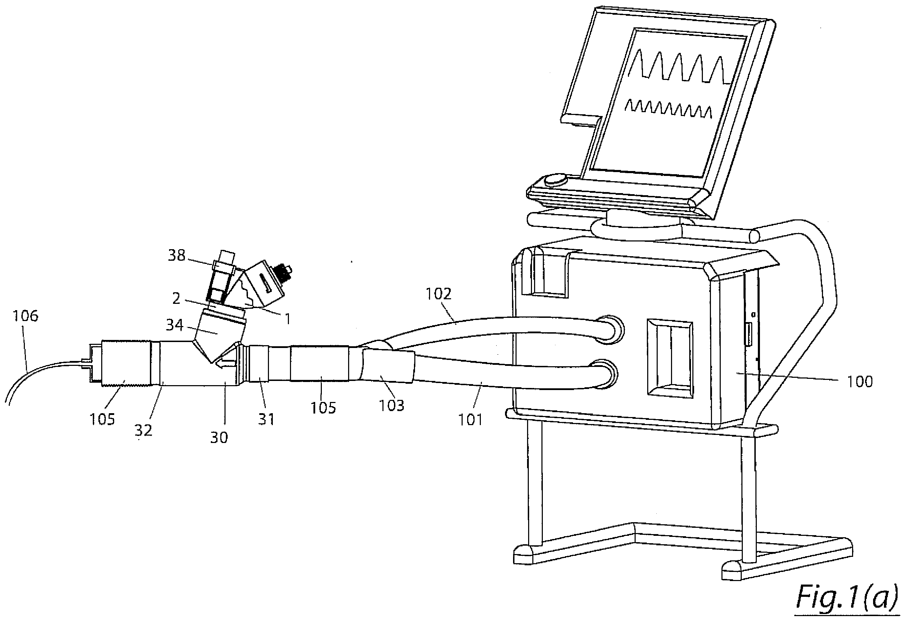

The humidifying system of the invention is particularly useful in delivering the aerosolised humidifying agent to a patient whose breathing is being assisted by a ventilator 100 as illustrated diagrammatically in Fig. 1A. An inhalation or inspiration line 101 extends from the ventilator 100. A return or exhalation line 102 also extends to the ventilator 100. The inspiration and exhalation lines are connected to a junction piece 103, which may be a wye junction. A patient line 105 extends from the wye 103 to an endotracheal tube 106 which extends to the patients lungs. Generally, the various lines 101, 102, 105, 106 are provided by lengths of plastic tubing which are interconnected. The tubing defines lumens for passage of ventilation air, during the inspiration phase, along the inspiration line 101, patient lines 105 and endotracheal tubes 106 into the patients lungs. During the expiration phase exhaled air is delivered along the endotracheal tube 106, patient lines 105 and the expiration line 102. The wye junction 103 provides a common pathway for inspiration and exhalation between the junction 103 and the patients lungs. The ventilator 100 mechanically assists the flow of oxygenated air to the patient during the inspiration phase and in the exhalation phase a patient exhales, either naturally or by the ventilator applying negative pressure.

The apparatus comprises a reservoir 1 for storing sterile water or saline solution, the aerosol generator 2 for aerosolising the water, and a controller 3 for controlling the operation of the aerosol generator 2.

In one aspect of the disclosure, an aerosol generator 2 is used to deliver an aerosolised humidifying agent into the ventilation air during the inspiration phase.

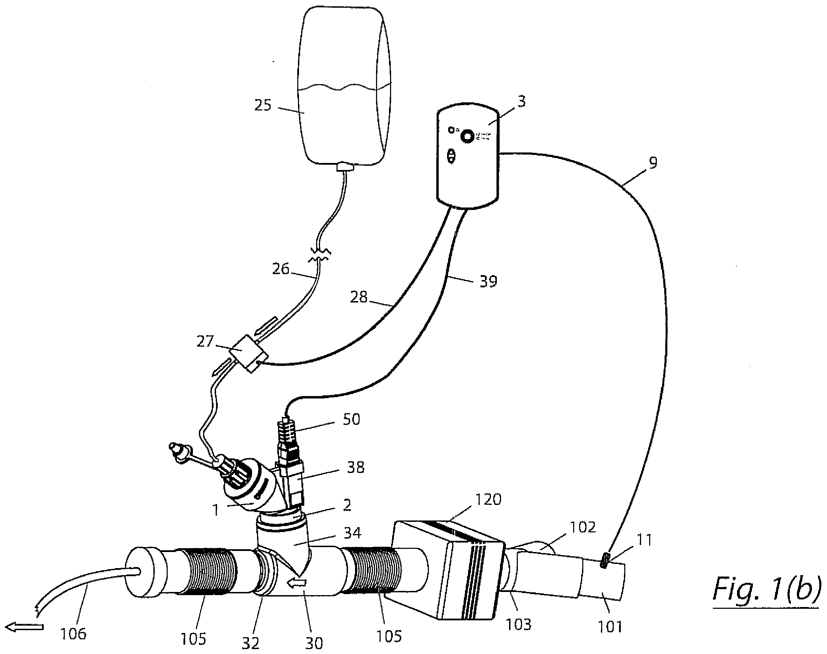

In the arrangement of Fig 1B the apparatus also comprises a sensor 11 for determining flow of air in the inspiration line 10. The sensor 11 is connected by a control wire 9 to the controller 3, and the aerosol generator 2 is also connected to the controller 3.

The humidifying agent may be sterile water or sterile saline with a salt concentration in the range from 1 micromolar to 154 millimolar. Such saline concentrations can be readily nebulised using the aerosolisation technology used in the invention.

An aerosol is delivered into the breathing circuit. The distinction between aerosol and vapour is in the size of the particles. The majority of aerosol particles that the aerosol generator produces are in the 0.5 to 5.0 micron diameter range. Water vapour on the other hand contains individual water molecules which are approximately 0.00001 microns i.e. 10,000 times smaller than the aerosol particles.

Medical gases for those patients on mechanical ventilation are humidified. The lung is conditioned to receive gas at close to 100% relative humidity (RH) .In the invention when undergoing mechanical ventilation the gas is also at 100% RH when exiting the endotracheal tube.

The amount of water a gas can hold is directly proportional to the temperature of the gas. The table below demonstrates the amount of water that air can hold at various temperatures to give 100% relative humidity

| **Air Temperature °C** | **Amount of H₂O required per L to give 100% RH** |
|---|---|
| 10 | 9.4 mg |
| 20 | 17.4 mg |
| 30 | 30.5 mg |
| 37 | 44.1 mg |

Thus, adding 0.044ml of H₂O to 1L of dry air at 37°C will result in the air having a relative humidity of 100%, making it suitable for patients undergoing mechanical ventilation.

This aerosol generator 2 converts the water into an aerosol of a very definable particle size. The volume mean diameter (vmd) would typically be in the range of 2 - 10 microns.

The controller 3 is used to provide electrical power to drive the aerosol generator. This provides the aerosolising action to convey humidification to the breathing circuit.

Referring to Fig. 1A and 1B, in this case an aerosol generator is placed between the Wye 103 and the endotracheal tube (ET) 106 to the patient. The aerosol generator is used to generate an aerosol of sterile water or sterile saline to humidify the gas being delivered to the patient.

In the arrangement of Fig. 1B 100% humidity at the end of the ET tube is achieved by having the nebulizer separate from the HME acting to top-up (boost) the humidity that is lost when using passive humidification via a HME.

In the case of an HME booster the aerosol generator 2 is placed between the ET and a HME 120 as illustrated in Fig.1B.

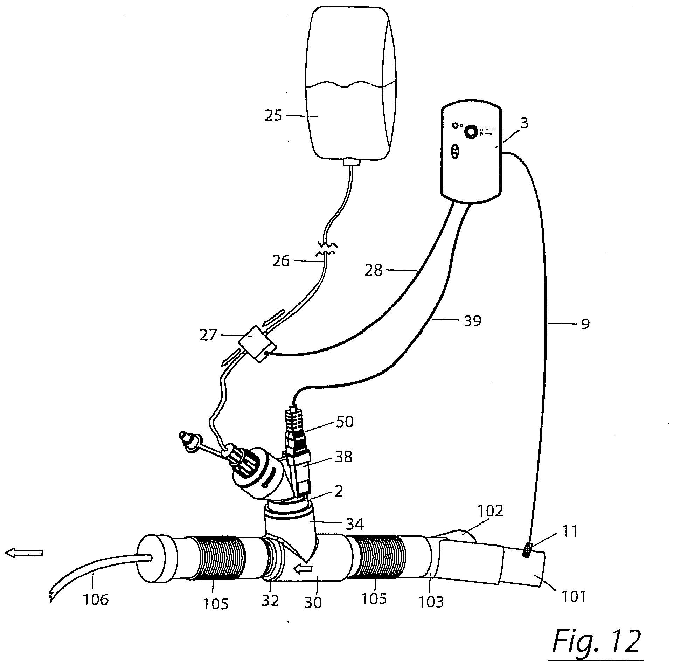

For non-boosting applications such as active humidification a HME unit is not required and an aerosol generator 2 is placed between the wye junction 103 and the endotracheal tube 106 as illustrated in Fig. 12. In the arrangement of Fig. 12 100% humidity at the end of the ET tube 106 is achieved because all the humidity for the patient is provided by the nebuliser 2 with no passive humidification.

Aerosol can be delivered continuously, intermittently in short bursts or generated only on inspiration. A flow meter (sensor) 11 may be placed in the inspiratory tubing 101 so that the aerosol output can be adjusted to the inspiratory flow. This provides feedback to the controller 3 to provide aerosol while the sensor 11 detects flow to the patient which occurs in the inhaled breath. Sterile water or sterile normal saline is used as the humidifying agent and the system is sealed from the atmosphere reducing contamination risk.

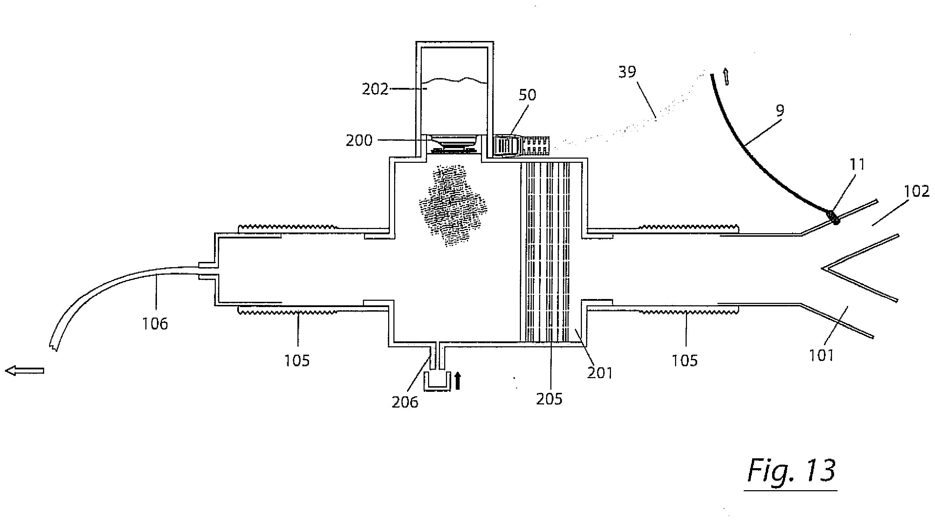

Another variant, in accordance with the present invention, is illustrated in Fig. 13. This shows a combination of an aerosol generator 200 and a HME (Heat and Moisture Exchange) filter 201 that has an inbuilt liquid reservoir 202. This functions as a booster for passive humidification. In the arrangement of Fig 13 100% humidity at the end of the ET tube is achieved by providing a top-up (boost) in humidity when using passive humidification via the HME, which is provided by an in-built aerosol generator 200 comprising a vibratable aperture plate which is incorporated into the HME. The HME unit 201 has a hydrophobic membrane 205 and is provided with a drain 206. The HME unit also has a drain - off port closable by a lid.

The in built liquid reservoir 202 may be replenished as required, so as to ensure an adequate supply of additional humidity to the circuit.

Aqueous solution may be stored in the reservoir 1 of the nebuliser or the aqueous solution may be delivered to the reservoir 1 of the aerosol generator 2 in this case from a supply reservoir 25 along a delivery line 26. The flow of aqueous solution may be by gravity and/or may be assisted by an in-line flow controlling device 27 such as a pump and/or a valve which may be positioned in the delivery line 26. The operation of the flow controlling device 27 may be controlled by the controller 3 along a control wire 28 to ensure that the aerosol generator 2 has a supply of aqueous solution during operation and yet does not allow fluid build up which may affect the operation of the aersoliser. The device 27 may be of any suitable type.

The vibrating mesh aerosol generator can work with many types of micro pumps. Flow rates of pumps depend on the application and aerosol output requirements however they are typically in the range of 50 nano litres per minute to 5 millilitres per minute. Such micro pumps can have different means of providing the pumping action and can include membrane pumps, electrohydrodynamic (EHD) pumps, electrokinetic, (EK) pumps, rotary pumps, peristaltic pumps, phase change pumps, and several other types of pumps. Diaphragm pumps that are driven by piezo activation are of particular interest as much of the control circuitry utilised is similar to that used to drive vibrating mesh technology and therefore integration of the circuits is simpler and cheaper to undertake.

The invention allows for the nebulization of liquids with surface tensions lower than water. These solutions are nebulizable but due to the surface tension they leak through the aperture plate when left sitting on it. When dispersed onto the aperture plate in a controlled drop by drop fashion the issue of leakage through the aperture plate will not occur.

The device also facilitates nebulisation of solutions that are prone to frothing. The potential to dispense the solution onto the aperture plate in a controlled fashion will prevent the build up of the solution on the aperture plate and the tendency to froth will be eliminated.

The device will reduce unnecessary exposure of solutions that are prone to oxidation or that are light sensitive.

As the pump feed can be located directly behind the aerosol plate it will remove the current restriction of the liquid feed being dependent and will allow the creation of aerosol through 360°C orientation of the device.

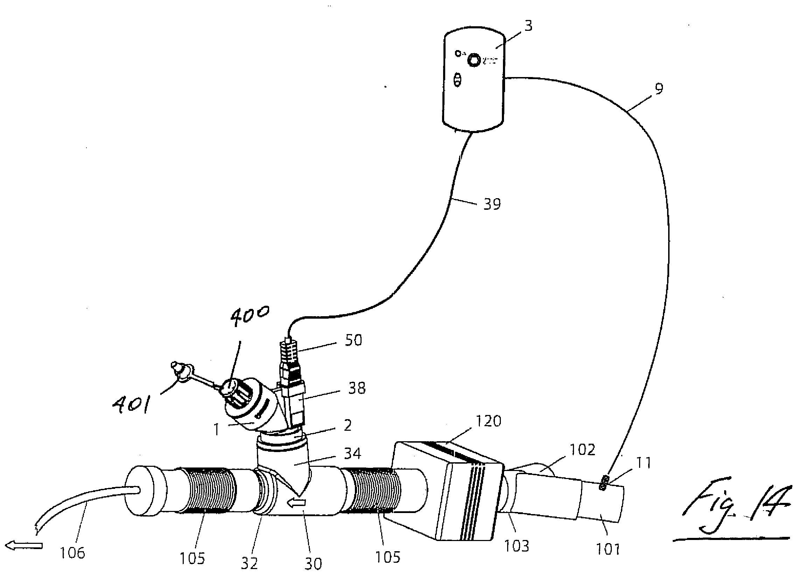

Referring to Fig. 14 there is illustrated another apparatus which is similar to that illustrated in Fig. 1B and like parts are assigned the same reference numerals. In this case the nebuliser reservoir 1 has a top opening 400 which is closable by removable plug 401. Liquid, saline or water for humidifying purposes and/or medicament is delivered into the nebuliser reservoir through the opening 400. The opening 400 is appropriately sized to receive standard nebulas containing liquid to be nebulised. The liquid may be applied by syringe or other suitable delivery means.

It is also possible to provide the nebuliser 1 pre-loaded with medicament to avoid the requirement to separately add medicament to the system.

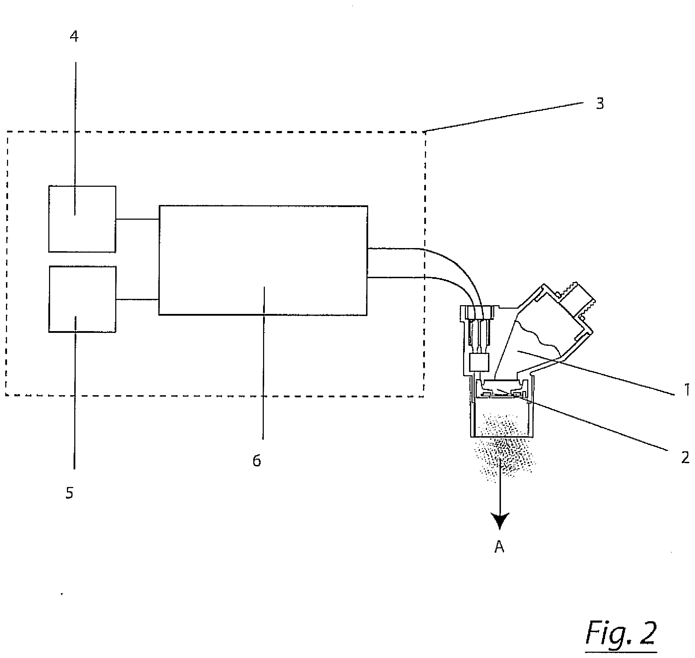

The apparatus of Fig. 14 is operated in a similar way to the modes of operation described with reference to Figs. 2 to 11.

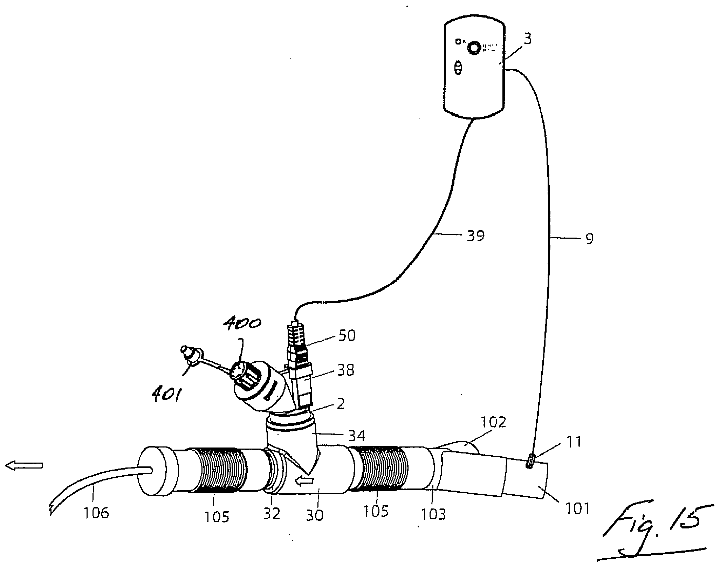

Referring to Fig. 15 there is illustrated another apparatus which is similar to that described above with reference to Fig. 12 and like parts are assigned the same reference numerals. In this case the nebuliser reservoir 1 has a top opening 400 and a removable plug/lid 401 as described with reference to Fig. 14 and the apparatus is operated as described above with the liquid being introduced through the opening 400. Again the nebuliser may be pre-loaded with medicament.

The apparatus comprises a connector 30, in this case a T-piece connector 30 having a ventilation gas conduit inlet 31 and an outlet 32. The connector 30 also comprises an aerosol supply conduit 34 for delivering the aerosol from the aerosol generator 2 into the gas conduit 105 to entrain the aerosol with the ventilation gas, passing through the gas conduit 105. The entrained aerosol/ventilation gas mixture passes out of the connector 30 through the outlet 32 and is delivered to the endotracheal tube 106.

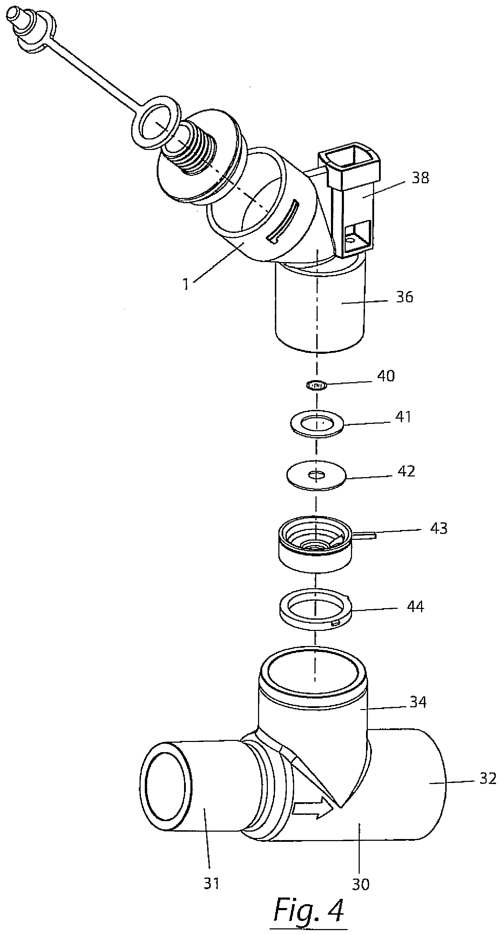

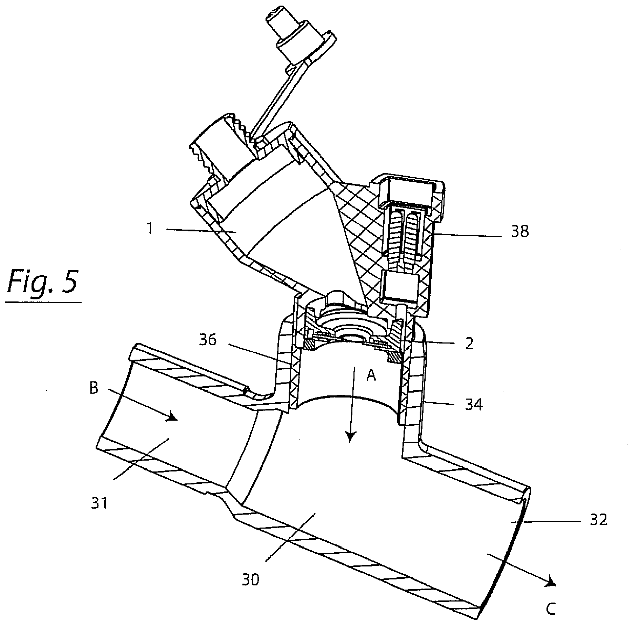

The aerosol supply conduit 34 and the ventilation gas conduit 105 meet at a junction. Referring particularly to Figs. 4 and 5, in the assembled apparatus the aerosol supply conduit of the connector 30 may be releasably mounted to a neck 36 of the aerosol generator housing by means of a push-fit arrangement. This enables the connector 30 to be easily dismounted from the aerosol generator housing 36, for example for cleaning. The neck 36 at least partially lines the interior of the aerosol supply conduit 34.

The nebuliser (or aerosol generator) 2, has a vibratable member which is vibrated at ultrasonic frequencies to produce liquid droplets. Some specific, non-limiting examples of technologies for producing fine liquid droplets is by supplying liquid to an aperture plate having a plurality of tapered apertures extending between a first surface and a second surface thereof and vibrating the aperture plate to eject liquid droplets through the apertures. Such technologies are described generally in U.S. Pat. Nos. 5,164,740; 5,938,117; 5,586,550; 5,758,637; 6,014,970, 6,085,740, and US2005/021766A. However, it should be appreciated that the present invention is not limited for use only with such devices.

Various methods of controlling the operation of such nebulisers or aerosol generators are described in US 6,540,154, US 6,845,770, US 5,938,117 and US 6,546,927.

In use, the liquid to be aerosolised is received at the first surface, and the aerosol generator 2 generates the aerosolised liquid at the second surface by ejecting droplets of the liquid upon vibration of the vibratable member. The apertures in the vibratable member are sized to aerosolise the liquid by ejecting droplets of the liquid such that the majority of the droplets by mass have a size of less than 5 micrometers.

Referring particularly to Figs 4 and 5, in one case the aerosol generator 2 comprises a vibratable member 40, a piezoelectric element 41 and a washer 42, which are sealed within a silicone overmould 43 and secured in place within the housing 36 using a retaining ring 44. The vibratable member 40 has a plurality of tapered apertures extending between a first surface and a second surface thereof.

The first surface of the vibratable member 40, which in use faces upwardly, receives the liquid from the reservoir 1 and the aerosolised liquid, is generated at the second surface of the vibratable member 40 by ejecting droplets of liquid upon vibration of the member 40. In use the second surface faces downwardly. In one case, the apertures in the vibratable member 40 may be sized to produce an aerosol in which the majority of the droplets by weight have a size of less than 5 micrometers.

The vibratable member 40 could be non-planar, and may be dome-shaped in geometry.

The complete nebuliser may be supplied in sterile form, which is a significant advantage for use in breathing circuits.

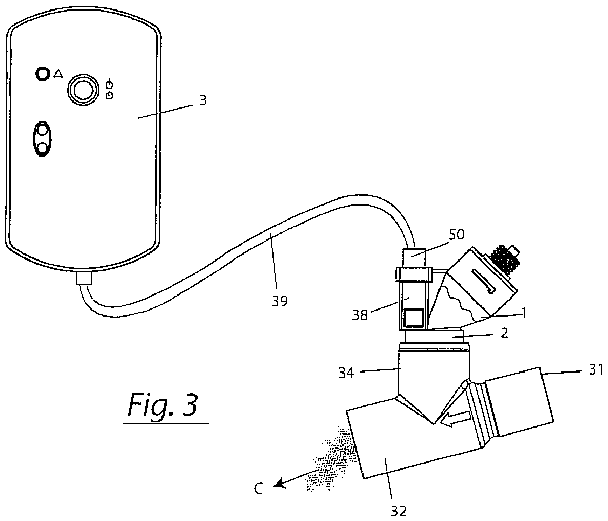

Referring particularly to Fig 3, the controller 3 controls operation of and provides a power supply to the aerosol generator 2. The aerosol generator has a housing which defines the reservoir 1. The housing has a signal interface port 38 fixed to the lower portion of the reservoir 1 to receive a control signal from the controller 3. The controller 3 may be connected to the signal interface port 38 by means of a control lead 39 which has a docking member 50 for mating with the port 38. A control signal and power may be passed from the controller 3 through the lead 39 and the port 38 to the aerosol generator 2 to control the operation of the aerosol generator 2 and to supply power to the aerosol generator 2 respectively.

The power source for the controller 3 may be an on-board power source, such as a rechargeable battery, or a remote power source, such as a mains power source, or an insufflator power source. When the remote power source is an AC mains power source, an AC-DC converter may be connected between the AC power source and the controller 3. A power connection lead may be provided to connect a power socket of the controller 3 with the remote power source.

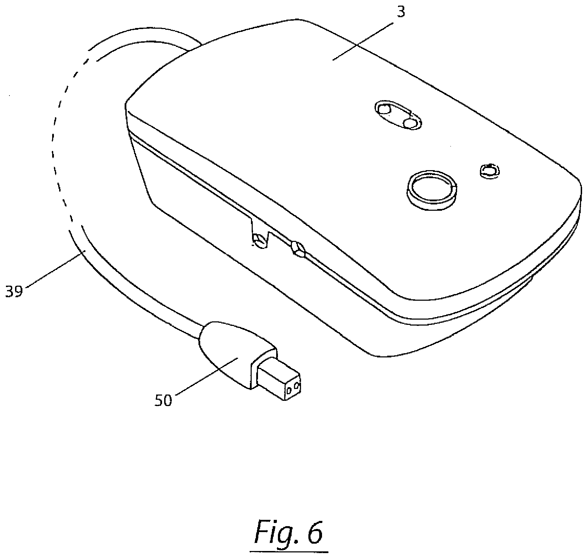

Referring particularly to Fig. 6 the controller 3 has a housing and a user interface to selectively control operation of the aerosol generator 2. Preferably the user interface is provided on the housing which, in use, is located remote from the aerosol generator housing. The user interface may be in the form of, for example, an on-off button. In one embodiment a button can be used to select pre-set values for simplicity of use. In another embodiment a dial mechanism can be used to select from a range of values from 0-100%.

Status indication means are also provided on the housing to indicate the operational state of the aerosol generator 2. For example, the status indication means may be in the form of two visible LED's, with one LED being used to indicate power and the other LED being used to indicate aerosol delivery. Alternatively one LED may be used to indicate an operational state of the aerosol generator 2, and the other LED may be used to indicate a rest state of the aerosol generator. 2.

A fault indicator may also be provided in the form of an LED on the housing. A battery charge indicator in the form of an LED may be provided at the side of the housing.

Referring particularly to Figs 1A and 1B, the liquid in the reservoir 1 flows by gravitational action towards the aerosol generator 2 at the lower medicament outlet. The controller 3 may then be activated to supply power and a control signal to the aerosol generator 2, which causes the piezoelectric element 41 to vibrate the non-planar member 40. This vibration of the non-planar member 40, causes the aqueous solution at the top surface of the member 40 to pass through the apertures to the lower surface where the aqueous solution is aerosolised by the ejection of small droplets of solution.

Referring particularly to Figs 4 and 5, the aerosol passes from the aerosol generator 2 into the neck 36 of the aerosol generator housing, which is mounted within the aerosol supply conduit of the connector 30 and into the gas conduit of the connector 30 (flow A). The aerosol is entrained in the ventilation gas conduit with gas, which passes into the gas conduit through the inlet 31 (flow B). The entrained mixture of the aerosol and the ventilation gas then passes out of the gas conduit through the outlet 32 (flow C) and on to the endotrachael tube 106.

The flow rate sensor/meter 11 determines the flow rate of the ventilation gas. In response to the fluid flow rate of the ventilation gas, the controller 3 commences operation of the aerosol generator 2 to aerosolise the aqueous solution. The aerosolised aqueous solution is entrained with the ventilation gas, and delivered to the patient.

In the event of alteration of the fluid flow rate of the ventilation gas, the flow rate sensor/meter 11 determines the alteration, and the controller 3 alters the pulse rate of the vibratable member of the nebuliser accordingly.

The controller 3 is in communication with the flow rate sensor/meter 11. The controller 3 is configured to control operation of the aerosol generator 2, responsive to the fluid flow rate of the ventilation gas and also independent of the fluid flow rate of the ventilation gas as required.

In one case, the controller 3 is configured to control operation of the aerosol generator 2 by controlling the pulse rate at a set frequency of vibration of the vibratable member, and thus controlling the fluid flow rate of the aqueous solutions.

The controller 3 may comprise a microprocessor 4, a boost circuit 5, and a drive circuit 6. Fig. 2 illustrates the microprocessor 4, the boost circuit 5, the drive circuit 6 comprising impedance matching components (inductor), the nebuliser 2, and the aerosol. The inductor impedance is matched to the impedance of the piezoelectric element of the aerosol generator 2. The microprocessor 4 generates a square waveform of 128KHz which is sent to the drive circuit 6. The boost circuit 5 generates a 12V DC voltage required by the drive circuit 6 from an input of either a 4.5V battery or a 9V AC/DC adapter. The circuit is matched to the impedance of the piezo ceramic element to ensure enhanced energy transfer. A drive frequency of 128 KHz is generated to drive the nebuliser at close to its resonant frequency so that enough amplitude is generated to break off droplets and produce the aerosol. If this frequency is chopped at a lower frequency such that aerosol is generated for a short time and then stopped for a short time this gives good control of the nebuliser's flow rate. This lower frequency is called the pulse rate.

The drive frequency may be started and stopped as required using the microprocessor 4. This allows for control of flow rate by driving the nebuliser 2 for any required pulse rate. The microprocessor 4 may control the on and off times to an accuracy of milliseconds.

The nebuliser 2 may be calibrated at a certain pulse rate by measuring how long it takes to deliver a know quantity of solution. There is a linear relationship between the pulse rate and the nebuliser flow rate. This may allow for accurate control over the delivery rate of the aqueous solution.

The nebuliser drive circuit consists of the electronic components designed to generate output sine waveform of approximately 100V AC which is fed to nebuliser 2 causing aerosol to be generated. The nebuliser drive circuit 6 uses inputs from microprocessor 4 and boost circuit 5 to achieve its output. The circuit is matched to the impedance of the piezo ceramic element to ensure good energy transfer.

The aerosol generator 2 may be configured to operate in a variety of different modes, such as continuous, and/or phasic, and/or optimised.

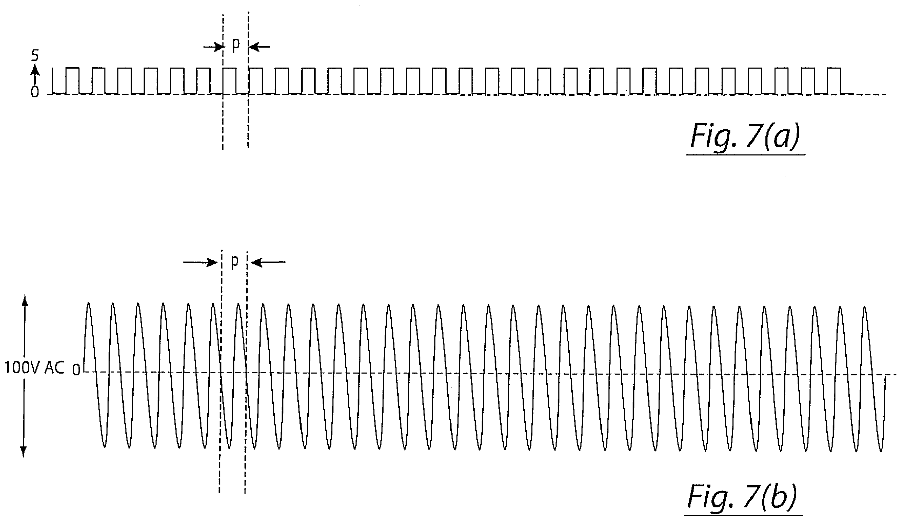

For example, referring to Fig 7(a) illustrates a 5V DC square waveform output from the microprocessor 4 to the drive circuit 6. Fig 7(b) shows a low power, ∼100V AC sine waveform output from drive circuit 6 to nebuliser 2. Both waveforms have a period p of 7.8µS giving them a frequency of 1/7.8µs which is approximately 128KHz. Both waveforms are continuous without any pulsing. The aerosol generator may be operated in this mode to achieve 100% aerosol output.

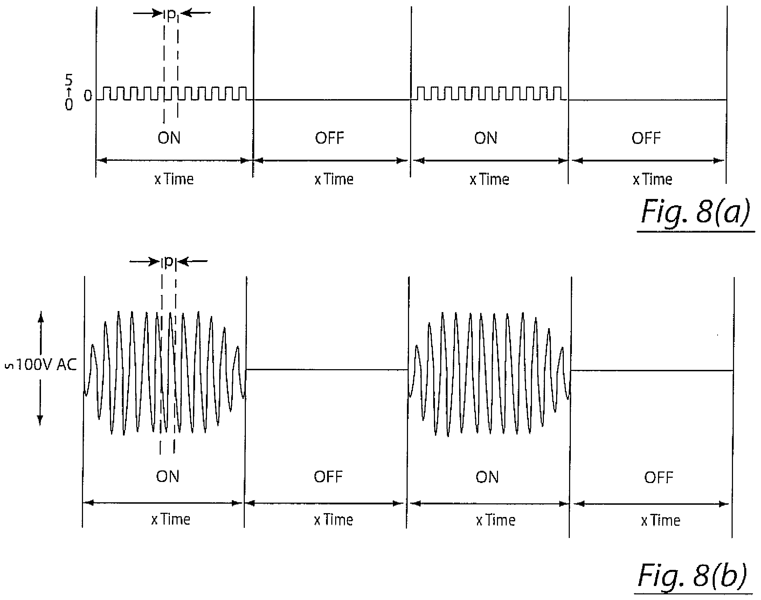

Referring to Figs 8(a) in another example, there is illustrated a 5V DC square waveform output from the microprocessor 4 to the drive circuit 6. Fig 8(b) shows a low power, ∼100V AC sine waveform output from the drive circuit 6 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time x is equal to the on time x. The aerosol generator may be operated in this mode to achieve 50% aerosol output.

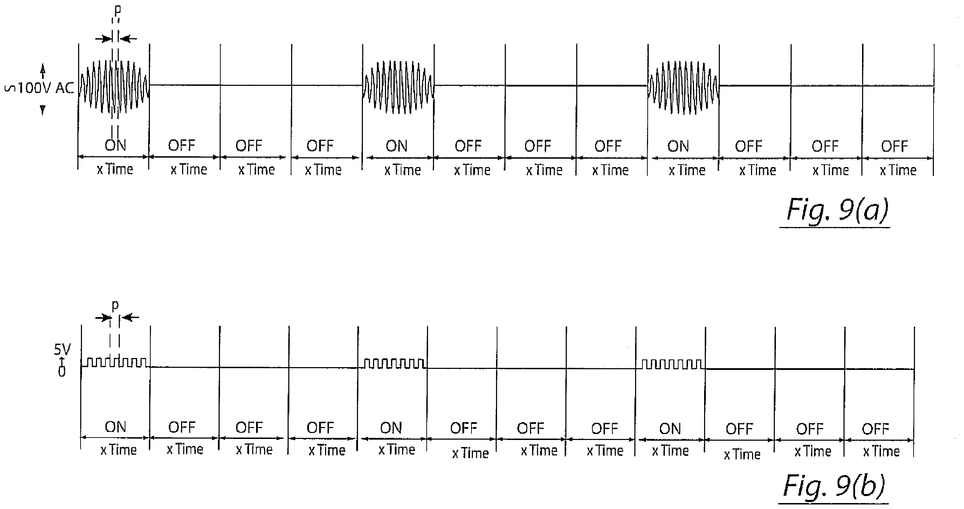

In another case, referring to Figs 9(a) there is illustrated a 5V DC square waveform output from microprocessor 4 to drive circuit 6. Fig 9(b) shows a low power, ∼100V AC sine waveform output from the drive circuit 6 to the nebuliser 2. Both waveforms have a period p of 7.8µS giving them a frequency of 1/7.8µs which is approximately 128KHz. In both cases the wavefoms are chopped (stopped/OFF) for a period of time x. In this case the off time is 3x while the on time is x. The aerosol generator may be operated in this mode to achieve 25% aerosol output.

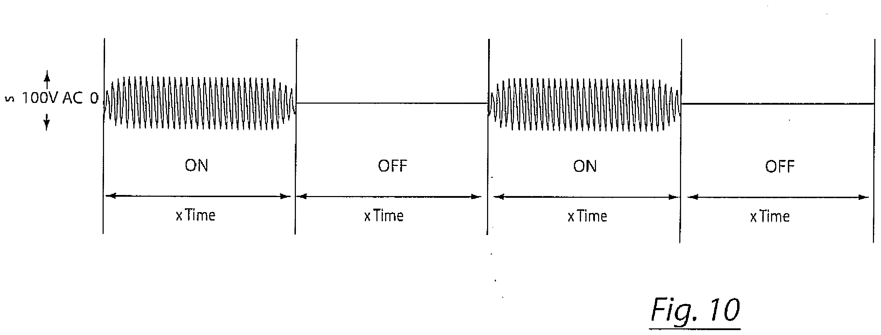

Referring to Fig 10, in one application pulsing is achieved by specifying an on-time and off-time for the vibration of the aperture plate. If the on-time is set to 200 vibrations and off-time is set to 200 vibrations, the pulse rate is 50% (½ on ½ off). This means that the flow rate is half of that of a fully driven aperture plate. Any number of vibrations can be specified but to achieve a linear relationship between flow rate and pulse rate a minimum number of on-time vibrations is specified since it takes a finite amount of time for the aperture plate to reach its maximum amplitude of vibrations.

The drive frequency can be started and stopped as required by the microprocessor; this allows control of flow rate by driving the nebuliser for any required pulse rate. The microprocessor can control the on and off times with an accuracy of microseconds.

A nebuliser can be calibrated at a certain pulse rate by measuring how long it takes to deliver a known quantity of solution. There is a linear relationship between the pulse rate and that nebuliser's flow rate. This allows accurate control of the rate of delivery of the aerosolised aqueous solution.

The pulse rate may be lowered so that the velocity of the emerging aerosol is much reduced so that impaction rain-out is reduced.

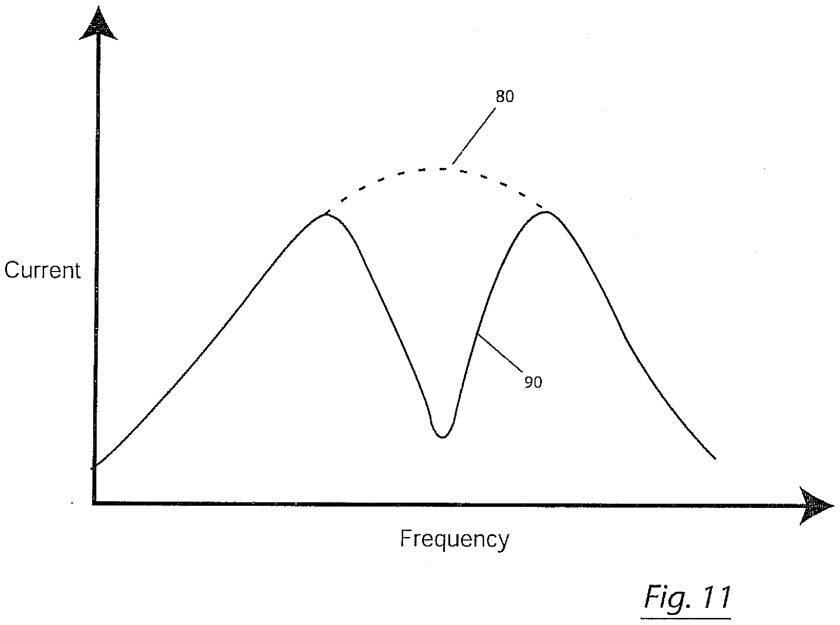

Detection of when the aperture plate is dry can be achieved by using the fact that a dry aperture plate has a well defined resonant frequency. If the drive frequency is swept from 120kHz to 145kHz and the current is measured then if a minimum current is detected less than a set value, the aperture plate must have gone dry. A wet aperture plate has no resonant frequency. The apparatus of the invention may be configured to determine whether there is any of the first fluid in contact with the aerosol generator 2. By determining an electrical characteristic of the aerosol generator 2, for example the current flowing through the aerosol generator 2, over a range of vibration frequencies, and comparing this electrical characteristic against a pre-defined set of data, it is possible to determine whether the aerosol generator 2 has any solution in contact with the aerosol generator 2. Fig. 11 illustrates a curve 80 of frequency versus current when there is some of the solution in contact with the aerosol generator 2, and illustrates a curve 90 of frequency versus current when there is none of the solution in contact with the aerosol generator 2. Fig. 11 illustrates the wet aperture plate curve 80 and the dry aperture plate curve 90.

If an application requires a constant feed from a drip bag then a pump can be added in line to give fine control of the liquid delivery rate which can be nebulised drip by drip. The rate would be set so that liquid would not build up in the nebuliser. This system is particularly suitable for constant low dose delivery.

In the invention the aerosol generator is placed at the patient's endotracheal tube so there is little to no rain - out in the tubing.

The device is very light and unlike the full heated wire system and very silent unlike the jet nebulizer. Non - heated single patient use ventilator tubing can be used. These bring considerable benefits:
▪ reduced cost of maintenance (care giver time)
▪ reduced heat/power cost (in excess of 10 fold)
▪ reduced cost of capital equipment and circuits
▪ reduced background noise

The supply to the aerosol generator is sealed from the atmosphere as it is in a closed circuit and so minimises infection risk even though the aerosol particle size is large enough to carry bacteria.

Intermittent short bursts of aerosol can be programmed to optimize water and heat replenishment of the HME, without requiring more complex aerosol generation patterns.

A drip feed line fed into a small volume reservoir allows the nebuliser to work in almost any orientation, reducing work and risk for the care giver. This also can provide a very low weight, low profile device.

With the aerosol used to augment the HME, another nebulizer for medication delivery can be placed between the HME and the patient ET, as described for example in US2005/0139211A.

The invention can be applied to systems used to ventilate all patients requiring mechanical ventilation or having bypassed upper airways requiring supplemental humidification.

All patients on mechanical ventilation require humidification either with a heated wire humidifier or a heat moisture exchanger. This system can be configured to add supplemental humidity to a HME (heat moisture exchange) only system thereby increasing the capacity of the system to adequately humidify patients for longer periods of time than is possible with current embodiments. The system can also be configured to fully replace the humidification element of a heated wire humidifier system by adding sufficient amounts of aerosol to the inspired air.

A major problem with the use of nebulizers in the past was contamination of the patient. This has generally been ascribed to the fact that the aerosol particles are of sufficient size to carry bacteria whereas vapour particles are not. The fact that the Aerogen nebulizers can be sterilized and also have the capacity to have a continuous feed of sterile liquid will overcome this reported disadvantage.

The key advantageous features of the invention are:
▪ small/compact
▪ quiet
▪ fed from a sterile sealed system
▪ no large power supply
▪ lower cost
▪ position independent operation

The invention is not limited to the embodiments hereinbefore described, which may be varied in construction and detail.

## Claims

1. A ventilator circuit apparatus comprising an endotracheal tube (106), an inspiration line (101) extending from a ventilator (100), and an exhalation line (102) extending from a ventilator, and an aerosol introducer apparatus for introducing aerosolised humidifying agent into the ventilation circuit, the introducer apparatus comprising an aerosol generator (2, 200) and control means for controlling the operation of the aerosol generator (2, 200) wherein the aerosol generator (2, 200) comprises a vibratable member (40) having a plurality of apertures extending between a first surface which is adapted to receive an agent to be aerosolised and a second surface at which an aerosol is generated wherein the ventilation circuit further comprises a junction (103) for connecting the inspiration line (101) and the exhalation line (102) and a patient line (105) for extending between the junction (103) and an endotracheal tube (106), **characterised in that** the ventilation circuit comprises a heat and moisture exchange unit (201) for location in the patient line (105), wherein the aerosol generator (2, 200) is arranged for delivery of aerosolised humidifying agent into the patient line (105) between the heat and moisture exchange unit (201) and the endotracheal tube (106), and wherein the aerosol generator (200) is mounted to the heat and moisture exchange unit (201).

2. An apparatus as claimed in claim 1 wherein the controller is configured to control operation of the aerosol generator (2, 200) responsive to the flow of gas in the inspiration line (101).

3. An apparatus as claimed in claim 1 or 2 wherein the controller is configured to control the flow rate of the humidifying agent to be aerosolised.

4. An apparatus as claimed in any of claims 1 to 3 wherein the apparatus comprises a device to determine the fluid flow rate of the gas in the inspiration line (101).

5. An apparatus as claimed in claim 4 wherein the determining device comprises a flow rate sensor (11).

6. An apparatus as claimed in claim 4 wherein the determining device comprises a differential pressure sensor (11).

7. An apparatus as claimed in any of claims 1 to 6 wherein the aerosol generator (2, 200) is integral with the heat and moisture exchange unit (201).

8. An apparatus as claimed in any of claims 1 to 7 wherein the heat and moisture exchange unit has a drain-off port (206).

9. An apparatus as claimed in any of claims 1 to 8 wherein the apertures in the vibratable member are sized:-
to aerosolise the humidifying agent by ejecting droplets such that the majority of the droplets by mass have a size of less than 5 micrometers;
to aerosolise the humidifying agent by ejecting droplets such that the majority of the droplets by mass have a size of less than 3 micrometers; or
to aerosolise the humidifying agent by ejecting droplets of the first fluid such that the majority of the droplets by mass have a size range of less than 10 micrometers, a range band is optionally from 1 to 3 micrometers and/or optionally from 7 to 9 micrometers.

10. An apparatus as claimed in any of claims 1 to 9 wherein the controller is configured to control the pulse rate at a set frequency of vibration of the vibratable member

11. An apparatus as claimed in any of claims 1 to 9 wherein the controller is impedance matched to the aerosol generator (2, 200).

12. An apparatus as claimed in any of claims 1 toll wherein the apparatus comprises means to determine whether the humidifying agent is in contact with the aerosol generator (2, 200).

13. An apparatus as claimed in claim 12 wherein the determining means is configured to determine at least one electrical characteristic of the aerosol generator (2, 200), the determining means is optionally configured to determine at least one electrical characteristic of the aerosol generator over a range of vibration frequencies.

14. An apparatus as claimed in claim 13 wherein the determining means is configured to compare the at least one electrical characteristic against a pre-defined set of data.

15. An apparatus as claimed in any of claims 1 to 14 comprising a flow controlling device for delivery of fluid to be aerosolised to the aerosol generator (2, 200).

16. An apparatus as claimed in claim 15 wherein the flow controlling device comprises a micropump, the micropump may comprise a diaphragm pump, optionally the diaphragm pump is driven by piezo activation.

17. Apparatus as claimed in claim 15 wherein the flow controlling device comprises a microvalve, the valve may be a solenoid valve.

18. Apparatus as claimed in any of claims 15 to 17 wherein the controller controls the operation of the flow controlling device.

## Patentansprüche

1. Beatmungsgerätkreislaufvorrichtung, umfassend einen Endotrachealtubus (106), eine sich von einem Beatmungsgerät (100) erstreckende Inspirationsleitung (101) und eine sich von einem Beatmungsgerät erstreckende Exhalationsleitung (102) und eine Aerosoleinbringungsvorrichtung zum Einbringen von aerosolisiertem Befeuchtungsmittel in den Beatmungskreislauf, wobei die Einbringungsvorrichtung einen Aerosolerzeuger (2, 200) und ein Steuermittel zum Steuern des Betriebs des Aerosolerzeugers (2, 200) umfasst, wobei der Aerosolerzeuger (2, 200) ein schwingungsfähiges Element (40) mit einer Vielzahl von sich zwischen einer ersten Oberfläche, die zum Aufnehmen eines zu aerosolisierenden Wirkstoffs angepasst ist, und einer zweiten Oberfläche, an der ein Aerosol erzeugt wird, erstreckenden Löchern umfasst, wobei der Beatmungskreislauf weiter eine Verzweigungsstelle (103) zum Anschließen der Inspirationsleitung (101) und der Exhalationsleitung (102) und eine Patientenleitung (105), um sich zwischen der Verzweigungsstelle (103) und einem Endotrachealtubus (106) zu erstrecken, umfasst, **dadurch gekennzeichnet, dass** der Beatmungskreislauf eine Wärme- und Feuchtigkeitsaustauscheinheit (201) zum Platzieren in der Patientenleitung (105) umfasst, wobei der Aerosolerzeuger (2, 200) zum Zuführen von aerosolisiertem Befeuchtungsmittel in die Patientenleitung (105) zwischen der Wärme- und Feuchtigkeitsaustauscheinheit (201) und dem Endotrachealtubus (106) angeordnet ist, und wobei der Aerosolerzeuger (200) an der Wärme- und Feuchtigkeitsaustauscheinheit (201) angebracht ist.

2. Vorrichtung nach Anspruch 1, wobei das Steuergerät dazu konfiguriert ist, den Betrieb des Aerosolerzeugers (2, 200) ansprechend auf den Strom von Gas in der Inspirationsleitung (101) zu steuern.

3. Vorrichtung nach einem Anspruch 1 oder 2, wobei das Steuergerät dazu konfiguriert ist, den Durchfluss des zu aerosolisierenden Befeuchtungsmittels zu steuern.

4. Vorrichtung nach einem der Ansprüche 1 bis 3, wobei die Vorrichtung eine Einrichtung zum Bestimmen des Fluiddurchflusses des Gases in der Inspirationsleitung (101) umfasst.

5. Vorrichtung nach Anspruch 4, wobei die Bestimmungseinrichtung einen Durchflusssensor (11) umfasst.

6. Vorrichtung nach Anspruch 4, wobei die Bestimmungseinrichtung einen Differenzdrucksensor (11) umfasst.

7. Vorrichtung nach einem der Ansprüche 1 bis 6, wobei der Aerosolerzeuger (2, 200) mit der Wärme- und Feuchtigkeitsaustauscheinheit (201) integriert ist.

8. Vorrichtung nach einem der Ansprüche 1 bis 7, wobei die Wärme- und Feuchtigkeitsaustauscheinheit einen Ableitungsanschluss (206) aufweist.

9. Vorrichtung nach einem der Ansprüche 1 bis 8, wobei die Löcher in dem schwingungsfähigen Element bemessen sind, um:
das Befeuchtungsmittel durch Ausstoßen von Tröpfchen zu aerosolisieren, sodass, bezogen auf die Masse, der Großteil der Tröpfchen eine Größe von weniger als 5 Mikrometern aufweist;
das Befeuchtungsmittel durch Ausstoßen von Tröpfchen zu aerosolisieren, sodass, bezogen auf die Masse, der Großteil der Tröpfchen eine Größe von weniger als 3 Mikrometern aufweist; oder
das Befeuchtungsmittel durch Ausstoßen von Tröpfchen des ersten Fluids zu aerosolisieren, sodass, bezogen auf die Masse, der Großteil der Tröpfchen einen Größenbereich von weniger als 10 Mikrometern aufweist, wobei ein Bereichsband optional von 1 bis 3 Mikrometern und/oder optional von 7 bis 9 Mikrometern beträgt.

10. Vorrichtung nach einem der Ansprüche 1 bis 9, wobei das Steuergerät dazu konfiguriert ist, die Impulsrate auf eine vorgegebene Frequenz der Schwingung des schwingungsfähigen Elements zu steuern.

11. Vorrichtung nach einem der Ansprüche 1 bis 9, wobei das Steuergerät an den Aerosolerzeuger (2, 200) impedanzangeapsst ist.

12. Vorrichtung nach einem der Ansprüche 1 bis 11, wobei die Vorrichtung ein Mittel umfasst, um zu bestimmen, ob sich das Befeuchtungsmittel mit dem Aerosolerzeuger (2, 200) in Kontakt befindet.

13. Vorrichtung nach Anspruch 12, wobei das Bestimmungsmittel dazu konfiguriert ist, mindestens eine elektrische Eigenschaft des Aerosolerzeugers (2, 200) zu bestimmen, wobei das Bestimmungsmittel optional dazu konfiguriert ist, mindestens eine elektrische Eigenschaft des Aerosolerzeugers über einen Bereich von Schwingungsfrequenzen hinweg zu bestimmen.

14. Vorrichtung nach Anspruch 13, wobei das Bestimmungsmittel dazu konfiguriert ist, die mindestens eine elektrische Eigenschaft mit einem vordefinierten Satz von Daten zu vergleichen.

15. Vorrichtung nach einem der Ansprüche 1 bis 14, umfassend eine Strömungssteuerungseinrichtung für die Zufuhr von zu aerosolisierendem Fluid zu dem Aerosolerzeuger (2, 200).

16. Vorrichtung nach Anspruch 15, wobei die Strömungssteuerungseinrichtung eine Mikropumpe umfasst, wobei die Mikropumpe eine Membranpumpe umfassen kann, wobei optional die Membranpumpe durch Piezoaktivierung angetrieben wird.

17. Vorrichtung nach Anspruch 15, wobei die Strömungssteuerungseinrichtung ein Mikroventil umfasst, wobei es sich bei dem Ventil um ein Magnetventil handeln kann.

18. Vorrichtung nach einem der Ansprüche 15 bis 17, wobei das Steuergerät den Betrieb der Strömungssteuerungseinrichtung steuert.

## Revendications

1. Appareil de circuit de ventilateur comprenant un tube endotrachéal (106), une ligne d'inspiration (101) s'étendant depuis un ventilateur (100), et une ligne d'expiration (102) s'étendant depuis un ventilateur, et un appareil introducteur d'aérosol pour introduire de l'agent humidifiant transformé en aérosol jusque dans le circuit de ventilation, l'appareil introducteur comprenant un générateur d'aérosol (2, 200) et un moyen de commande pour commander le fonctionnement du générateur d'aérosol (2, 200) dans lequel le générateur d'aérosol (2, 200) comprend un élément capable de vibrer (40) ayant une pluralité d'ouvertures s'étendant entre une première surface qui est conçue pour recevoir un agent devant être transformé en aérosol et une deuxième surface au niveau de laquelle un aérosol est généré dans lequel le circuit de ventilation comprend en outre un branchement (103) pour connecter la ligne d'inspiration (101) et la ligne d'expiration (102) et une ligne de patient (105) destinée à s'étendre entre le branchement (103) et un tube endotrachéal (106), **caractérisé en ce que** le circuit de ventilation comprend une unité échangeuse de chaleur et d'humidité (201) destinée à être située dans la ligne de patient (105), dans lequel le générateur d'aérosol (2, 200) est agencé pour distribuer de l'agent humidifiant transformé en aérosol jusque dans la ligne de patient (105) entre l'unité échangeuse de chaleur et d'humidité (201) et le tube endotrachéal (106), et dans lequel le générateur d'aérosol (200) est monté sur l'unité échangeuse de chaleur et d'humidité (201).

2. Appareil selon la revendication 1 dans lequel le dispositif de commande est configuré pour commander le fonctionnement du générateur d'aérosol (2, 200) en réponse à l'écoulement de gaz dans la ligne d'inspiration (101).

3. Appareil selon la revendication 1 ou 2 dans lequel le dispositif de commande est configuré pour commander le débit de l'agent humidifiant devant être transformé en aérosol.

4. Appareil selon l'une quelconque des revendications 1 à 3 dans lequel l'appareil comprend un dispositif pour déterminer le débit fluidique du gaz dans la ligne d'inspiration (101).

5. Appareil selon la revendication 4 dans lequel le dispositif de détermination comprend un détecteur de débit (11).

6. Appareil selon la revendication 4 dans lequel le dispositif de détermination comprend un détecteur de pression différentielle (11).

7. Appareil selon l'une quelconque des revendications 1 à 6 dans lequel le générateur d'aérosol (2, 200) est formé d'un seul tenant avec l'unité échangeuse de chaleur et d'humidité (201).

8. Appareil selon l'une quelconque des revendications 1 à 7 dans lequel l'unité échangeuse de chaleur et d'humidité a un orifice d'égouttage (206).

9. Appareil selon l'une quelconque des revendications 1 à 8 dans lequel les ouvertures dans l'élément capable de vibrer sont dimensionnées :
pour transformer en aérosol l'agent humidifiant en éjectant des gouttelettes de sorte que la majorité des gouttelettes en masse ont une taille inférieure à 5 micromètres ;
pour transformer en aérosol l'agent humidifiant en éjectant des gouttelettes de sorte que la majorité des gouttelettes en masse ont une taille inférieure à 3 micromètres ; ou
pour transformer en aérosol l'agent humidifiant en éjectant des gouttelettes du premier fluide de sorte que la majorité des gouttelettes en masse ont une plage de tailles inférieure à 10 micromètres, une bande de plage est facultativement de 1 à 3 micromètres et/ou facultativement de 7 à 9 micromètres.

10. Appareil selon l'une quelconque des revendications 1 à 9 dans lequel le dispositif de commande est configuré pour commander le taux d'impulsions à une fréquence réglée de vibration de l'élément capable de vibrer.

11. Appareil selon l'une quelconque des revendications 1 à 9 dans lequel le dispositif de commande est adapté en impédance au générateur d'aérosol (2, 200).

12. Appareil selon l'une quelconque des revendications 1 à 11 dans lequel l'appareil comprend un moyen pour déterminer si l'agent humidifiant est en contact avec le générateur d'aérosol (2, 200).

13. Appareil selon la revendication 12 dans lequel le moyen de détermination est configuré pour déterminer au moins une caractéristique électrique du générateur d'aérosol (2, 200), le moyen de détermination est facultativement configuré pour déterminer au moins une caractéristique électrique du générateur d'aérosol sur une plage de fréquences de vibration.

14. Appareil selon la revendication 13 dans lequel le moyen de détermination est configuré pour comparer l'au moins une caractéristique électrique avec une série prédéfinie de données.

15. Appareil selon l'une quelconque des revendications 1 à 14 comprenant un dispositif de régulation de débit pour distribuer du fluide devant être transformé en aérosol vers le générateur d'aérosol (2, 200).

16. Appareil selon la revendication 15 dans lequel le dispositif de régulation d'écoulement comprend une micropompe, la micropompe peut comprendre une pompe à diaphragme, facultativement la pompe à diaphragme est entraînée par une activation piézoélectrique.

17. Appareil selon la revendication 15 dans lequel le dispositif de régulation d'écoulement comprend une microvanne, la vanne peut être une électrovanne.

18. Appareil selon l'une quelconque des revendications 15 à 17 dans lequel le dispositif de commande commande le fonctionnement du dispositif de régulation d'écoulement.
